# EUROPEAN PATENT APPLICATION

(11) **EP 4 759 778 A1**
(43) Date of publication of application: **17.06.2026**
(21) Application number: 24851356.6
(22) Date of filing: 21.05.2024
(51) Int. Cl.: C03C 3/066, A61K 6/77, A61K 6/836

(54) **GLASS**

(30) Priority: 10.08.2023 JP 2023131549
(71) Applicant: Sumita Optical Glass, Inc., Saitama-shi, Saitama 330-8565 (JP)
(72) Inventor: ARAKI, Mao, Saitama-shi, Saitama 330-8565 (JP); SASAKI, Ryoichi, Kurashiki-shi Okayama 713-8550 (JP); SHIMIZU, Satoki, Tainai-shi Niigata 959-2653 (JP); KUDO, Yasutaka, Tainai-shi Niigata 959-2653 (JP); TERAKAWA, Eiichi, Tainai-shi Niigata 959-2653 (JP)
(74) Representative: Scott, Stephen John
(86) International application number: PCT/JP2024/018740
(87) International publication number: WO 2025/032922

(57) **Abstract**

Provided is a glass that simultaneously achieves both chemical durability and sufficient acid neutralizing capacity and that is suitable as a dental composition displaying transparency when combined with a resin. The glass has a composition containing, by mass%, not less than 5.00% and not more than 15.00% of Si, not less than 2.00% and less than 6.00% of Al, not less than 10.00% and not more than 30.00% of Sr, not less than 2.00% and not more than 25.00% of Zn, not less than 2.00% and not more than 10.00% of B, not less than 17.00% and not more than 30.00% of F, not less than 16.00% and not more than 40.00% of O, and not less than 0% and not more than 0.50%, in total, of Li, Na, and K.

## Description

### TECHNICAL FIELD

The present disclosure relates to a glass.

### BACKGROUND

Dental composite materials (hereinafter, also referred to as composite materials) that contain a resin and an inorganic filler are often used instead of conventional metal materials in the treatment of tooth decay or loss. Such composite materials excel in terms of mechanical strength, durability, and the like, which makes them useful for restoring the shape of a target site and as adhesives for adhering dental materials to teeth.

In restorative treatment using such composite materials, there is demand for transparency that is as close as possible to natural tooth structure from an aesthetic viewpoint. In order to achieve such transparency through combination of a resin and an inorganic filler, it is important for the refractive index of the resin and the refractive index of the inorganic filler to be of the same level.

When the inside of an oral cavity becomes acidic due to consumption of food and beverages, attachment of plaque (food debris, detached mucosa, etc.), or the like, this results in demineralization in which calcium ions and the like elute from the tooth structure and acts as a cause of tooth decay. Although the acid neutralizing capacity of saliva normally maintains the inside of the oral cavity at a neutral pH of around 7, there is a limit to this neutralizing capacity. Therefore, it is also desirable for an inorganic filler such as mentioned above to display neutralizing capacity in acidic environments.

As one example of a dental filler having a refractive index that is harmonized with that of a resin, PTL 1 discloses a glass that contains Si, Al, K, Zr, and so forth and that has a refractive index (nd) of not less than 1.518 and not more than 1.533.

However, the dental glass described in PTL 1 does not function to inhibit demineralization and has poor acid neutralizing capacity, which means that there is a high likelihood of tooth decay arising.

On the other hand, PTL 2 discloses a composition in which a fluoroaluminosilicate glass containing Si, Al, B, Na, and Sr is used. This composition is expected to provide improved acid resistance of tooth structure through release of divalent to tetravalent ions from the fluoroaluminosilicate glass, acid neutralizing effect inside of an oral cavity, and improved acid resistance of tooth structure through release of fluoride ions.

### CITATION LIST

### Patent Literature

PTL 1: JP 2010-189261 A
PTL 2: JP 2015-227309 A

### SUMMARY

### (Technical Problem)

However, although the glass used in the composition described in PTL 2 can display sufficient acid neutralizing capacity, chemical durability has been an issue with this glass.

The present disclosure was developed in light of the circumstances set forth above and an object thereof is to provide a glass that simultaneously achieves both chemical durability and sufficient acid neutralizing capacity and that is suitable as a dental composition displaying transparency when combined with a resin.

### (Solution to Problem)

As a result of extensive diligent studies conducted to achieve the object described above, the inventors discovered that by adopting specific amounts of Si, Al, B, O, and F as a basic composition, by optimizing the contents of Sr and Zn, and by restricting the content of one or more of Li, Na, and K to 0.50% or less, it is possible to obtain a glass that simultaneously achieves both chemical durability and sufficient acid neutralizing capacity and that is suitable as a dental composition displaying transparency when combined with a resin.

Specifically, a glass according to the present disclosure comprises a composition containing, by mass%:
not less than 5.00% and not more than 15.00% of Si;
not less than 2.00% and less than 6.00% of Al;
not less than 10.00% and not more than 30.00% of Sr;
not less than 2.00% and not more than 25.00% of Zn;
not less than 2.00% and not more than 10.00% of B;
not less than 17.00% and not more than 30.00% of F;
not less than 16.00% and not more than 40.00% of O; and
not less than 0% and not more than 0.50%, in total, of Li, Na, and K.

This glass simultaneously achieves both chemical durability and sufficient acid neutralizing capacity and displays transparency when combined with a resin.

With the glass according to the present disclosure, it is preferable that when a powder of the glass is added into pH 3.6 acetic acid aqueous solution of 37°C such as to have a concentration of 20 mass% to yield a glass mixture and is subjected to 1 hour of immersion, the glass mixture has a pH of 4.4 or higher after the 1 hour of immersion, and weight loss of the powder of the glass due to the 1 hour of immersion is 0.40 mass% or less.

The glass according to the present disclosure preferably has a d line refractive index (nd) of 1.500 to 1.600.

### (Advantageous Effect)

According to the present disclosure, it is possible to provide a glass that simultaneously achieves both chemical durability and sufficient acid neutralizing capacity and that displays transparency when combined with a resin.

### DETAILED DESCRIPTION

The following provides a specific description of the present disclosure using embodiments.

### (Glass)

A glass according to one embodiment of the present disclosure (hereinafter, also referred to as the "glass of the present embodiment") has a composition containing, by mass%:
not less than 5.00% and not more than 15.00% of Si;
not less than 2.00% and less than 6.00% of Al;
not less than 10.00% and not more than 30.00% of Sr;
not less than 2.00% and not more than 25.00% of Zn;
not less than 2.00% and not more than 10.00% of B;
not less than 17.00% and not more than 30.00% of F;
not less than 16.00% and not more than 40.00% of O; and
not less than 0% and not more than 0.50%, in total, of Li, Na, and K.

The glass of the present embodiment may contain other components (described further below) besides the components described above (Si, Al, Sr, Zn, B, F, O, Li, Na, and K). However, from a viewpoint of more reliably causing the expression of the desired acid neutralizing capacity, sufficient chemical durability, and transparency upon combination with a resin, the glass of the present embodiment preferably has a composition consisting of only the components described above.

The phrase "consisting of only the components described above" as used here encompasses a case in which impurity components other than the above-described components are unavoidably contained, and, more specifically, encompasses a case in which the proportion constituted by such impurity components is 0.20 mass% or less.

First, reasons for limiting the composition of the glass to the ranges set forth above in the present embodiment are described. Note that "%" used in relation to components means mass% unless otherwise specified.

### <Si>

Si is an essential component in the glass of the present embodiment and is a main component that forms a network structure serving as a framework of the glass. Moreover, Si is a component that can increase devitrification resistance and strength of the glass. However, when the content of Si in the glass is more than 15.00%, meltability decreases. On the other hand, when the content of Si in the glass is less than 5.00%, devitrification resistance cannot be sufficiently obtained. Therefore, the content of Si in the glass of the present embodiment is set as a range of not less than 5.00% and not more than 15.00%. From a similar viewpoint, the content of Si is preferably 6.00% or more, and more preferably 7.00% or more, and is preferably 14.00% or less, and more preferably 13.00% or less. SiO₂, Al₂Si₂O₅(OH)₄, Zn₂SiO₄, or the like, for example, may be used as a material corresponding to Si, and one of these materials may be used individually, or two or more of these materials may be used in combination.

### <Al>

Al is an essential component in the glass of the present embodiment and is a component that through addition together with Sr, etc., can form a network structure of the glass, improve devitrification resistance, and lower the refractive index. However, Al has been found to conversely decrease acid neutralizing capacity. When the content of Al in the glass is more than 6.00%, acid neutralizing capacity and chemical durability of the glass decrease. On the other hand, when the content of Al in the glass is less than 2.00%, the refractive index of the glass rises. Therefore, the content of Al in the glass of the present embodiment is set as a range of not less than 2.00% and less than 6.00%. From a similar viewpoint, the content of Al is preferably 2.50% or more, and more preferably 3.00% or more, and is preferably 5.50% or less, and more preferably 5.00% or less. Al₂O₃, Al₂Si₂O₅(OH)₄, AlF₃, or the like, for example, may be used as a material corresponding to Al, and one of these materials may be used individually, or two or more of these materials may be used in combination.

### <Sr>

Sr is an essential component in the glass of the present embodiment and is a component that can increase acid neutralizing capacity. Moreover, Sr is a component that can increase radiopacity. However, when the content of Sr in the glass is more than 30.00%, devitrification resistance decreases. On the other hand, when the content of Sr in the glass is less than 10.00%, acid neutralizing capacity cannot be sufficiently obtained. Therefore, the content of Sr in the glass of the present embodiment is set as a range of not less than 10.00% and not more than 30.00%. From a similar viewpoint, the content of Sr is preferably 11.00% or more, and more preferably 12.00% or more, and is preferably 29.00% or less, and more preferably 28.00% or less. SrCO₃, SrF₃, or the like, for example, may be used as a material corresponding to Sr, and one of these materials may be used individually, or two or more of these materials may be used in combination.

### <Zn>

Zn is an essential component in the glass of the present embodiment and is a component that can increase chemical durability of the glass. Moreover, Zn is a component that can increase radiopacity. However, when the content of Zn in the glass is more than 25.00%, the refractive index of the glass rises. On the other hand, when the content of Zn in the glass is less than 2.00%, chemical durability of the glass cannot be sufficiently obtained. Therefore, the content of Zn in the glass of the present embodiment is set as a range of not less than 2.00% and not more than 25.00%. From a similar viewpoint, the content of Zn is preferably 3.00% or more, and more preferably 4.00% or more, and is preferably 24.00% or less, and more preferably 23.00% or less. ZnO, ZnF₂, or the like, for example, may be used as a material corresponding to Zn, and one of these materials may be used individually, or two or more of these materials may be used in combination.

### <B>

B is an essential component in the glass of the present embodiment and is a main component that forms a network structure serving as a framework of the glass. Moreover, B is a component that can increase devitrification resistance and meltability of the glass. However, when the content of B in the glass is more than 10.00%, chemical durability of the glass decreases. On the other hand, when the content of B in the glass is less than 2.00%, devitrification resistance cannot be sufficiently obtained. Therefore, the content of B in the glass of the present embodiment is set as a range of not less than 2.00% and not more than 10.00%. From a similar viewpoint, the content of B is preferably 3.00% or more, and more preferably 4.00% or more, and is preferably 9.00% or less, and more preferably 8.00% or less. B₂O₃, H₃BO₃, or the like, for example, may be used as a material corresponding to B, and one of these materials may be used individually, or two or more of these materials may be used in combination.

### <F>

F is an essential component in the glass of the present embodiment and is a component that can increase chemical durability of the glass. Moreover, the release of fluoride ions can be expected to improve acid resistance of tooth structure. However, when the content of F in the glass is more than 30.00%, devitrification resistance decreases. On the other hand, when the content of F in the glass is less than 17.00%, chemical durability of the glass cannot be sufficiently obtained. Therefore, the content of F in the glass of the present embodiment is set as a range of not less than 17.00% and not more than 30.00%. From a similar viewpoint, the content of F is preferably 18.00% or more, and more preferably 19.00% or more, and is preferably 29.00% or less, and more preferably 28.00% or less. AlF₃, SrF₂, ZnF₂, NH₄F, or the like, for example, may be used as a material corresponding to F, and one of these materials may be used individually, or two or more of these materials may be used in combination.

### <O>

O is an essential component in the glass of the present embodiment and is a main component that forms a network structure serving as a framework of the glass. Moreover, O is a component that can improve devitrification resistance. However, when the content of O in the glass is more than 40.00%, acid neutralizing capacity cannot be sufficiently obtained. On the other hand, when the content of O in the glass is less than 16.00%, devitrification resistance decreases. Therefore, the content of O in the glass of the present embodiment is set as a range of not less than 16.00% and not more than 40.00%. From a similar viewpoint, the content of O is preferably 17.00% or more, more preferably 18.00% or more, even more preferably 20.00% or more, and further preferably 22.00% or more, and is preferably 39.00% or less, more preferably 38.00% or less, even more preferably 35.00% or less, and further preferably 32.00% or less. Various oxides such as SiO₂, B₂O₃, and Al₂O₃, for example, may be used as a material corresponding to O, and one of these materials may be used individually, or two or more of these materials may be used in combination.

### <Li, Na, K>

Li, Na, and K are optional components in the glass of the present embodiment. These components are components that improve meltability of the glass. However, when the total content of Li, Na, and K in the glass is more than 0.50%, devitrification resistance cannot be sufficiently obtained. Therefore, the total content of Li, Na, and K in the glass of the present embodiment is set as a range of 0.50% or less. From a similar viewpoint, the total content of Li, Na, and K is preferably 0.40% or less, and more preferably 0.30% or less. Moreover, the content of each component among Li, Na, and K is preferably 0.35% or less, and more preferably 0.30% or less. Li₂CO₃, LiF, or the like, for example, may be used as a material corresponding to Li. Na₂CO₃, NaF, Na₃AlF₆, or the like, for example, may be used as a material corresponding to Na. K₂CO₃, KF, K₂SiF₆, or the like, for example, may be used as a material corresponding to K. One of these materials may be used individually, or two or more of these materials may be used in combination.

### <Other components>

The glass of the present embodiment can contain other components besides the above-described components so long as this does not result in deviation from the object. For example, the glass of the present embodiment can contain a small amount of Mg, Ca, Ba, P, Sc, Ti, Y, Zr, Ta, La, Ce, Pr, Nd, Sm, Eu, Gd, Tb, Dy, Ho, Er, Tm, Yb, Lu, etc. (for example, an amount such that the total of these other components is 3 mass% or less). Note that the glass of the present embodiment preferably does not contain components such as Cr, Co, Ni, Pb, Te, As, and Cd that have a high environmental impact or negative effect on the human body.

Next, various characteristics of the glass of the present embodiment are described.

### <Refractive index (nd)>

In order to ensure transparency, it is preferable that the refractive index of the glass of the present embodiment is of roughly the same level as the refractive index of a resin formed by adding a polymerization initiator to a polymerizable monomer to cure the polymerizable monomer. Although hydroxyapatite, which is a main component of natural teeth, has a refractive index of approximately 1.6, the apparent refractive index of natural teeth, which have a composite structure, is slightly lower. Therefore, the use of a dental composite material having a refractive index adjusted to approximately 1.500 to 1.600 is considered to result in a natural appearance. Note that the refractive index of the resin is determined by the polymerizable monomer. For example, a d line refractive index (nd) of not less than 1.500 and not more than 1.600 can be obtained by mixing BMHPE, UDMA, POBMA, etc. in specific proportions and performing curing thereof. Accordingly, the glass of the present embodiment preferably has a d line refractive index (nd) of not less than 1.500 and not more than 1.600 in order to be of the same level as the refractive index of the resin.

Note that the refractive index (nd) can be measured by a procedure described in the EXAMPLES section. Also note that adjustment of the refractive index (nd) of the glass of the present embodiment can be performed through appropriate adjustment of the contents of the above-described components within specific ranges, for example.

### <pH after acidic solution immersion>

The glass of the present embodiment preferably has a property of raising the pH through a neutralization reaction in an acidic environment in order to prevent tooth decay. More specifically, with the glass of the present embodiment, it is preferable that when a powder of the glass is added into pH 3.6 acetic acid aqueous solution of 37°C such as to have a concentration of 20 mass% to yield a glass mixture and is subjected to 1 hour of immersion, the glass mixture has a pH of 4.4 or higher after the 1 hour of immersion.

Note that the pH after acidic solution immersion can be measured by a procedure described in the EXAMPLES section. Also note that adjustment of the pH after acidic solution immersion can be performed through appropriate adjustment of the contents of the above-described components within specific ranges, for example.

### <Glass sample weight loss after acidic solution immersion>

The glass of the present embodiment preferably has a small glass sample weight loss in an acidic environment, which serves as an indicator of good chemical durability. More specifically, with the glass of the present embodiment, it is preferable that when a powder of the glass is added into pH 3.6 acetic acid aqueous solution of 37°C such as to have a concentration of 20 mass% to yield a glass mixture and is subjected to 1 hour of immersion, weight loss of the powder of the glass after the 1 hour of immersion is 0.40 mass% or less.

Note that the glass sample weight loss after acidic solution immersion can be measured by a procedure described in the EXAMPLES section. Also note that adjustment of the glass sample weight loss after acidic solution immersion can be performed through appropriate adjustment of the contents of the above-described components within specific ranges, for example.

### <Production method of glass>

Next, the production method of the glass of the present embodiment is described.

The glass of the present embodiment can be produced according to a conventional production method without any specific limitations on the production method thereof so long as the composition of components satisfies the ranges set forth above.

For example, oxides, fluorides, hydroxides, carbonates, nitrates, etc. are first weighed out in specific proportions as materials for components that can be contained in the glass of the present embodiment and are thoroughly mixed to obtain a glass batch. Next, this batch is loaded into a melting vessel (for example, a crucible made of a precious metal such as platinum) that is not reactive with the glass materials and is heated and melted at 1000°C to 1400°C. Thereafter, stirring is performed in a timely manner to promote homogenization and perform refining, and then rapid cooling is performed, thereby enabling production of the glass.

### <Application>

Since the glass of the present embodiment simultaneously achieves both chemical durability and sufficient acid neutralizing capacity and displays transparency when combined with a resin as described above, the glass of the present embodiment can be used in a dental composition (dental composite material), for example. In other words, the glass of the present embodiment may, for example, be dental glass. This dental composition may, for example, contain at least the glass of the present embodiment as an inorganic filler and a resin formed by adding a polymerization initiator to a polymerizable monomer to cure the polymerizable monomer.

The polymerizable monomer used to form the resin in the dental composition can be any of those that are commonly known as polymerizable monomers used in dental compositions. Examples include 1,2-bis(3-methacryloyloxy-2-hydroxypropoxy)ethane (BMHPE), 2,2,4-trimethylhexamethylenebis(2-carbamoyloxyethyl) dimethacrylate (UDMA), m-phenoxybenzyl methacrylate (POBMA), and so forth. One of these polymerizable monomers may be used individually, or two or more of these polymerizable monomers may be used in combination.

The polymerization initiator used to form the resin in the dental composition can be any of those that are commonly known as polymerization initiators used in dental compositions. Examples include camphorquinone (CQ), ethyl 4-N,N-dimethylaminobenzoate (PDE), and so forth. One of these polymerization initiators may be used individually, or two or more of these polymerization initiators may be used in combination.

The dental composition can be produced according to a conventional production method without any specific limitations on the production method thereof so long as the glass of the present embodiment is used.

For example, the glass of the present embodiment set forth above is first ground using a ball mill. Next, 100 parts by mass of the resultant ground glass is subjected to surface treatment by a standard method using 1.5 parts by mass of γ-methacryloyloxypropyltrimethoxysilane to thereby obtain silane treated glass powder. Next, this silane treated glass powder, BMHPE and UDMA serving as polymerizable monomers, and CQ and PDE serving as polymerization initiators are weighed out in specific proportions and are thoroughly mixed and kneaded at normal temperature to obtain a dental mixed paste. Next, this dental mixed paste is subjected to photoirradiation by a dental LED photoirradiation instrument so as to cure the paste, thereby enabling production of a dental composition containing the glass of the present embodiment and a resin.

### EXAMPLES

The following provides a more specific description of the present disclosure through examples and comparative examples. However, the present disclosure is not limited to these examples.

At least any one of an oxide, a fluoride, a carbonate, and a nitrate corresponding to each component was prepared as a material for that component. These materials were weighed out such that the composition after vitrification was as indicated in Tables 1 and 2 and were thoroughly mixed to obtain a batch. The batch was loaded into a platinum crucible and was melted in an electric furnace. Note that the temperature during melting was adjusted as appropriate within a range of 1000°C to 1400°C in order to promote sufficient bubble removal. Thereafter, stirring was performed in a timely manner to promote homogenization and perform refining, and then rapid cooling was performed to obtain a glass (glass could not be obtained in some cases). In addition, after melting and homogenization of a batch by the same procedure, the batch was cast into a mold that was preheated to an appropriate temperature and was then slowly cooled inside of an electric furnace to relieve strain and thereby obtain a glass for refractive index measurement.

The obtained glass of each example or comparative example was then subjected to measurement of refractive index (nd), pH after acidic solution immersion, and glass sample weight loss due to acidic solution immersion according to procedures described below. Note that these measurements were not performed in cases in which a glass could not be obtained as mentioned above. The results are shown in Table 1 and Table 2.

### <Refractive index>

Measurement of the d line refractive index (nd) was performed in accordance with a refractive index measurement method of JIS B 7071-2 using the glass for refractive index measurement described above. When the d line refractive index (nd) is not less than 1.500 and not more than 1.600, the glass is advantageous in terms of transparency when combined with a resin.

### <pH after acidic solution immersion>

The glass obtained by rapid cooling was ground in a mortar, was passed through a 30-mesh sieve, and a glass powder of a size retained at 40-mesh was obtained. After loading 1.0000 g of the glass powder into a test tube, 4.0 mL of pH 3.6 acetic acid aqueous solution was added to obtain a glass mixture (i.e., a mixture having a glass concentration of 20 mass%), and 1 hour of immersion was performed in a constant-temperature water tank set to 37°C. Thereafter, the test tube was removed from the constant-temperature water tank, 0.5 mL of a supernatant of the glass mixture was sampled, and the pH thereof was measured using a compact pH meter (LAQUAtwin produced by HORIBA, Ltd.). When the measured pH is 4.4 or higher, this indicates that the glass has good acid neutralizing capacity. Note that the pH meter was calibrated using pH 4.01 and pH 6.86 reference solutions prior to use.

### <Glass sample weight loss after acidic solution immersion>

Prior to the acidic solution immersion described above, the mass (M_{TC}) of the test tube by itself was recorded. The mass (M_{TC}) of the test tube by itself was subtracted from the mass (M_{TC+bS}) of the test tube after loading of the glass powder to thereby calculate the mass (M_{bS} = M_{TC+bS} - M_{TC}) of the glass powder (glass sample) prior to acidic solution immersion. Next, once loading of acetic acid aqueous solution and immersion had been performed, 5 mL of ethanol was added into the test tube after pH measurement, and the supernatant was removed by decantation. Supernatant removal by decantation was repeated three times, and then 1 hour of drying at 100°C was performed in a dryer. The mass (M_{TC}) of the test tube by itself was subtracted from the mass (M_{TC+aS}) of the test tube and the glass sample after drying to thereby calculate the mass (M_{aS} = M_{TC+aS} - M_{TC}) of the glass sample after acidic solution immersion. The glass sample weight loss after acidic solution immersion was then calculated by (M_{bS} - M_{aS})/M_{bS} × 100. When this calculated value is small, and particularly 0.40% or less, this indicates excellent chemical durability.

**[Table 1]**

| | | Example 1 | Example 2 | Example 3 | Example 4 | Example 5 | Example 6 | Example 7 | Example 8 | Example 9 | Example 10 |
|---|---|---|---|---|---|---|---|---|---|---|---|
| Si | [Mass%] | 11.22 | 11.98 | 11.22 | 11.22 | 9.82 | 11.22 | 12.57 | 11.69 | 7.01 | 6.08 |
| Al | | 3.21 | 2.36 | 4.91 | 5.98 | 5.14 | 5.78 | 2.47 | 4.82 | 4.82 | 5.46 |
| Sr | | 13.95 | 18.67 | 24.41 | 22.67 | 20.23 | 20.93 | 19.67 | 20.93 | 20.92 | 15.35 |
| Zn | | 18.97 | 13.91 | 6.96 | 6.96 | 3.79 | 6.96 | 11.00 | 3.16 | 6.33 | 16.07 |
| B | | 4.66 | 5.68 | 4.97 | 4.81 | 8.70 | 5.28 | 5.99 | 7.76 | 9.32 | 8.70 |
| F | | 23.86 | 21.16 | 22.77 | 23.71 | 21.84 | 25.33 | 20.38 | 21.09 | 22.93 | 18.19 |
| Ti | | 0.60 | 0.00 | 0.00 | 0.00 | 0.00 | 0.00 | 0.00 | 0.00 | 0.00 | 0.00 |
| Na | | 0.00 | 0.00 | 0.00 | 0.00 | 0.00 | 0.00 | 0.27 | 0.00 | 0.00 | 0.00 |
| O | | 23.53 | 26.24 | 24.76 | 24.65 | 30.48 | 24.50 | 27.65 | 30.55 | 28.67 | 30.15 |
| Total | | 100.00 | 100.00 | 100.00 | 100.00 | 100.00 | 100.00 | 100.00 | 100.00 | 100.00 | 100.00 |
| Li + Na + K | | 0.00 | 0.00 | 0.00 | 0.00 | 0.00 | 0.00 | 0.27 | 0.00 | 0.00 | 0.00 |
| pH after acidic solution immersion | | 4.9 | 5.0 | 5.0 | 4.7 | 4.8 | 4.6 | 4.8 | 4.6 | 4.8 | 4.8 |
| Glass sample weight loss after acidic solution immersion | [%] | 0.07 | 0.09 | 0.10 | 0.08 | 0.26 | 0.13 | 0.13 | 0.25 | 0.12 | 0.12 |
| Refractive index | | 1.598 | 1.594 | 1.566 | 1.568 | 1.558 | 1.563 | 1.580 | 1.552 | 1.560 | 1.541 |

**[Table 2]**

| | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 | Comparative Example 13 | Comparative Example 14 | Comparative Example 15 |
|---|---|---|---|---|---|---|---|---|---|---|---|---|---|---|---|---|
| Si | [Mass%] | 26.67 | 9.26 | 3.74 | 16.36 | 10.28 | 11.13 | 11.69 | 9.35 | 12.62 | 6.54 | 13.09 | 6.08 | 7.01 | 6.08 | 14.02 |
| Al | | 2.85 | 10.48 | 4.82 | 3.21 | 0.32 | 9.18 | 4.82 | 4.82 | 5.46 | 2.57 | 3.21 | 2.57 | 4.82 | *5.46* | 3.53 |
| Sr | | 0.00 | 29.60 | 26.63 | 13.95 | 20.93 | 19.93 | 9.07 | 31.39 | 19.53 | 13.95 | 22.70 | 20.93 | 11.20 | 18.83 | 14.65 |
| Zn | | 0.00 | 0.00 | 20.73 | 12.65 | 15.81 | 3.01 | 15.81 | 3.16 | 0.95 | 26.65 | 20.05 | 5.69 | 20.08 | 18.97 | 13.28 |
| B | | 3.44 | 3.63 | 4.04 | 4.66 | 6.83 | 4.44 | 6.83 | 4.66 | 8.23 | 6.52 | 0.93 | 12.42 | 9.32 | 4.04 | 4.66 |
| F | | 0.00 | 11.40 | 21.74 | 20.19 | 18.94 | 29.78 | 20.21 | 25.63 | 20.56 | 18.10 | 19.22 | 17.81 | 13.21 | 30.73 | 22.44 |
| Ti | | 0.00 | 0.00 | 0.00 | 0.00 | 0.00 | 0.00 | 0.00 | 0.00 | 0.00 | 0.00 | 0.21 | 0.00 | 0.00 | 0.00 | 0.00 |
| Na | | 2.00 | 1.71 | 0.00 | 0.00 | 0.00 | 0.00 | 0.00 | 0.00 | 0.00 | 0.00 | 0.00 | 0.00 | 0.00 | 0.00 | 1.10 |
| K | | 10.17 | 0.00 | 0.00 | 0.00 | 0.00 | 0.00 | 0.00 | 0.00 | 0.00 | 0.00 | 0.00 | 0.00 | 0.00 | 0.00 | 0.00 |
| La | | 4.88 | 0.00 | 0.00 | 0.00 | 0.00 | 0.00 | 0.00 | 0.00 | 0.00 | 0.00 | 0.00 | 0.00 | 0.00 | 0.00 | 0.00 |
| Zr | | 4.73 | 0.00 | 0.00 | 0.00 | 0.00 | 0.00 | 0.00 | 0.00 | 0.00 | 0.00 | 0.00 | 0.00 | 0.00 | 0.00 | 0.00 |
| O | | 45.26 | 33.92 | 18.30 | 28.98 | 26.89 | 22.53 | 31.57 | 20.99 | 32.65 | 25.67 | 20.59 | 34.50 | 34.36 | 15.89 | 26.32 |
| Total | | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |
| Li + Na + K | | 12.17 | 1.71 | 0.00 | 0.00 | 0.00 | 0.00 | 0.00 | 0.00 | 0.00 | 0.00 | 0.00 | 0.00 | 0.00 | 0.00 | 1.10 |
| pH after acidic solution immersion | | 4.1 | 4.7 | Glass not obtained | Glass not obtained | 5.4 | 4.1 | 4.1 | Glass not obtained | 4.6 | 5.2 | Glass not obtained | 4.9 | 5.2 | Glass not obtained | Glass not obtained |
| Glass sample weight loss after acidic solution immersion | [%] | 0.07 | 0.48 | | | 0.22 | 0.50 | 0.13 | | 0.49 | 0.24 | | 0.43 | 0.61 | | |
| Refractive index | | 1.526 | 1.530 | | | 1.621 | 1.540 | 1.533 | | 1.534 | 1.625 | | 1.557 | 1.610 | | |

It can be seen from Table 1 that the glasses of Examples 1 to 10 each result in a pH of 4.4 or higher after acetic acid aqueous solution immersion and have good acid neutralizing capacity. It can also be seen that the glasses of Examples 1 to 10 each result in a glass sample weight loss after acetic acid aqueous solution immersion of 0.40% or less and have sufficient chemical durability. Moreover, it can be seen that the glasses of Examples 1 to 10 each have a refractive index of not less than 1.500 and not more than 1.600 and are advantageous in terms of transparency when combined with a resin.

In contrast, it can be seen from Table 2 that in Comparative Example 1, although the glass sample weight loss after acidic solution immersion was small, the pH after acidic solution immersion was lower than pH 4.4.

In Comparative Example 2, although the pH after acidic solution immersion was 4.4 or higher, the glass sample weight loss after acidic solution immersion was more than 0.40%.

In Comparative Example 3, devitrification occurred and a glass could not be obtained. This may be due to insufficient Si, for example.

In Comparative Example 4, meltability was poor up to at least 1400°C, and hence devitrification occurred and a glass could not be obtained. This may be due to excessive Si, for example.

In Comparative Example 5, the refractive index was higher than 1.600. This may be due to insufficient Al, for example.

In Comparative Example 6, the pH was lower than 4.4. Moreover, the glass sample weight loss after acidic solution immersion was more than 0.40%. This may be due to excessive Al, for example.

In Comparative Example 7, the pH was lower than 4.4. This may be due to insufficient Sr, for example.

In Comparative Example 8, devitrification occurred and a glass could not be obtained. This may be due to excessive Sr, for example.

In Comparative Example 9, the glass sample weight loss after acidic solution immersion was more than 0.40%. This may be due to insufficient Zn, for example.

In Comparative Example 10, devitrification occurred and a glass could not be obtained. This may be due to excessive Zn, for example.

In Comparative Example 11, devitrification occurred and a glass could not be obtained. This may be due to insufficient B, for example.

In Comparative Example 12, the glass sample weight loss after acidic solution immersion was more than 0.40%. This may be due to excessive B, for example.

In Comparative Example 13, the glass sample weight loss after acidic solution immersion was more than 0.40%. This may be due to insufficient F, for example.

In Comparative Example 14, devitrification occurred and a glass could not be obtained. This may be due to insufficient F and O, for example.

In Comparative Example 15, devitrification occurred and a glass could not be obtained. This may be due to one or more of Li, Na, and K being excessive, for example.

### INDUSTRIAL APPLICABILITY

According to the present disclosure, it is possible to provide a glass that simultaneously achieves both chemical durability and sufficient acid neutralizing capacity and that displays transparency when combined with a resin.

## Claims

1. A glass comprising a composition containing, by mass%:
not less than 5.00% and not more than 15.00% of Si;
not less than 2.00% and less than 6.00% of Al;
not less than 10.00% and not more than 30.00% of Sr;
not less than 2.00% and not more than 25.00% of Zn;
not less than 2.00% and not more than 10.00% of B;
not less than 17.00% and not more than 30.00% of F;
not less than 16.00% and not more than 40.00% of O; and
not less than 0% and not more than 0.50%, in total, of Li, Na, and K.

2. The glass according to claim 1, wherein, when a powder of the glass is added into pH 3.6 acetic acid aqueous solution of 37°C such as to have a concentration of 20 mass% to yield a glass mixture and is subjected to 1 hour of immersion, the glass mixture has a pH of 4.4 or higher after the 1 hour of immersion, and weight loss of the powder of the glass due to the 1 hour of immersion is 0.40 mass% or less.

3. The glass according to claim 1 or 2, wherein the glass has a d line refractive index (nd) of 1.500 to 1.600.
